# EUROPEAN PATENT APPLICATION

(11) **EP 4 145 382 A1**
(43) Date of publication of application: **08.03.2023**
(21) Application number: 21813401.3
(22) Date of filing: 28.05.2021
(51) Int. Cl.: G06T 3/00

(54) **IMAGE PROCESSING METHOD AND SYSTEM**

(30) Priority: 28.05.2020 CN 202010469129
(71) Applicant: Shanghai United Imaging Healthcare Co., Ltd., Shanghai 201807 (CN)
(72) Inventor: LIU, Yanyan, Shanghai 201807 (CN); QUAN, Guotao, Shanghai 201807 (CN)
(74) Representative: Wang, Bo
(86) International application number: PCT/CN2021/096990
(87) International publication number: WO 2021/239141

(57) **Abstract**

Some embodiments of the present disclosure may relate to an image processing system and method. The image processing system and method may include: obtaining an initial image; determining, based on the initial image, a plurality of to-be-processed images related to the initial image; and processing the plurality of to-be-processed images based on a processing model to obtain a target image of the initial image.

## Description

### CROSS-REFERENCE TO RELATED APPLICATIONS

This application claims priority to Chinese Patent Application No. 202010469129.2 filed on May 28, 2020, the entire contents of each of which are incorporated herein by reference.

### TECHNICAL FIELD

The present disclosure relates to a technical field of image processing, and in particular, to methods and systems for processing medical images using a neural network model.

### BACKGROUND

Through analysis and processing of medical images, feature information of organs, soft tissues, lesions, etc., of an object (such as a human body) may be extracted, so as to assist an operator (such as a doctor) to perform a qualitative or quantitative analysis on lesion bodies and other areas of interest, improving the accuracy and reliability of medical diagnosis. For some current medical image analysis and processing methods, the processing speed or denoising effect is ordinary. Therefore, it is desirable to provide a system and a method for processing a medical image based on a neural network model, so as to improve the quality or processing speed of the medical image.

### SUMMARY

One aspect of the present disclosure may provide a system. The system may include at least one storage device including a set of instructions; and at least one processor in communication with the at least one storage device, wherein, when executing the set of instructions, the at least one processor may be configured to cause the system to perform operations including: obtaining an initial image; determining, based on the initial image, a plurality of to-be-processed images related to the initial image; and processing the plurality of to-be-processed images based on a processing model to obtain a target image of the initial image.

In some embodiments, the initial image may be a two-dimensional image, the plurality of to-be-processed images may include information of the initial image, or the target image may be a two-dimensional image.

In some embodiments, each of the plurality of to-be-processed images may be a two-dimensional image, or the plurality of to-be-processed images may correspond to a three-dimensional image.

In some embodiments, a similarity of structure information or texture information of regions of interest in at least two of the plurality of to-be-processed images may exceed a threshold.

In some embodiments, at least two of the plurality of to-be-processed images may be generated based on data acquired by imaging devices of different modalities.

In some embodiments, the target image may include identification information related to a region of interest, and the identification information related to the region of interest may include a contour of the region of interest, a location of the region of interest, or a size of the region of interest.

In some embodiments, the determining, based on the initial image, a plurality of to-be-processed images related to the initial image may include: obtaining at least two additional images; and determining the initial image and at least one of the at least two additional images as the plurality of to-be-processed images.

In some embodiments, the determining, based on the initial image, a plurality of to-be-processed images related to the initial image may include: selecting a plurality of consecutive images adjacent to the initial image as the plurality of to-be-processed images. In some embodiments, the determining, based on the initial image, a plurality of to-be-processed images related to the initial image may include: extracting a feature of the initial image; obtaining a plurality of image blocks based on the feature of the initial image, wherein at least two of the plurality of image blocks may include image regions with a same feature; and determining the plurality of to-be-processed images based on the plurality of image blocks.

In some embodiments, the determining the plurality of to-be-processed images based on the plurality of image blocks may include: determining the plurality of to-be-processed images by performing a matrix transformation on each of the plurality of image blocks.

In some embodiments, the processing model may include a neural network model.
In some embodiments, the neural network model may include a matrix transformation module, and the neural network model may be configured to: perform a three-dimensional convolution processing based on a plurality of two-dimensional images or a three-dimensional image;
perform a matrix transformation processing on a result of the three-dimensional convolution processing; perform a two-dimensional convolution processing on a result of the matrix transformation processing; and obtain a two-dimensional image by performing a linear processing on a result of the two-dimensional convolution processing.

In some embodiments, the processing the plurality of to-be-processed images based on a processing model to obtain a target image may include: inputting the plurality of to-be-processed images into the neural network model; obtaining a first processing result by performing a two-dimensional convolution processing on the plurality of to-be-processed images in a plurality of channels of the neural network model, respectively; obtaining a second processing result by performing a linear processing on the first processing result; and obtaining the target image based on the second processing result.

In some embodiments, the obtaining a first processing result by performing a two-dimensional convolution processing on the plurality of to-be-processed images in a plurality of channels of the neural network model, respectively, may include: in each of the plurality of channels of the neural network model, obtaining a corresponding portion of the first processing result by performing the two-dimensional convolution processing on one of the plurality of to-be-processed images.

In some embodiments, the plurality of to-be-processed images may include a plurality of image blocks, and the obtaining the target image based on the second processing result may include: fusing the second processing result to determine the target image, or fusing the second processing result and the initial image to determine the target image.

In some embodiments, the processing the plurality of to-be-processed images based on a processing model to obtain a target image may include: obtaining a third processing result by performing a three-dimensional convolution processing on the plurality of to-be-processed images; obtaining a plurality of dimension-reduced intermediate images corresponding to the plurality of to-be-processed images by performing a dimension-reduction processing on the third processing result; obtaining a fourth processing result by performing a two-dimension convolution processing on the plurality of dimension-reduced intermediate images in a plurality of channels of the neural network model, respectively; obtaining a fifth processing result by performing a linear processing on the fourth processing result; and obtaining the target image based on the fifth processing result.

In some embodiments, the initial image, the plurality of to-be-processed images, or the target image may include at least one of a computed tomography image, a nuclear magnetic resonance image, a positron emission computed tomography image, or an ultrasound image.

One aspect of the present disclosure may provide a method. The method may be implemented on a computing device including at least one processor and at least one storage device. The method may include: obtaining an initial image; determining, based on the initial image, a plurality of to-be-processed images related to the initial image; and processing the plurality of to-be-processed images based on a processing model to obtain a target image.

One aspect of the present disclosure may relate to a non-transitory computer-readable medium including executable instructions, wherein when executed by at least one processor, the executable instructions may direct the at least one processor to perform a method. The method may include: obtaining an initial image; determining, based on the initial image, a plurality of to-be-processed images related to the initial image; and processing the plurality of to-be-processed images based on a processing model to obtain a target image.

One aspect of the present disclosure may relate to a system. The system may include: an obtaining module configured to obtain an initial image; a determination module configured to determine, based on the initial image, a plurality of to-be-processed images related to the initial image; and a processing module configured to process the plurality of to-be-processed images based on a processing model to obtain a target image.

Some of the additional features of the present disclosure may be described in the following descriptions. Some of the additional features of the present disclosure may be apparent to those skilled in the art from a study of the following descriptions and the corresponding drawings or from a knowledge of the production or operation of the embodiments. The features of the present disclosure may be realized and attained through the practice or use of the methods, means, and combinations of the various aspects of the specific embodiments described below.

### BRIEF DESCRIPTION OF THE DRAWINGS

The present disclosure may be further described in terms of exemplary embodiments, which may be described in detail with reference to the drawings. The drawings are not drawn to scale. These embodiments are not limiting, and in these embodiments, the same reference numerals in the various drawings represent similar structures, and wherein:
FIG. 1 is a schematic diagram of an exemplary image processing system according to some embodiments of the present disclosure;
FIG. 2 is a schematic diagram of hardware and/or software components of an exemplary computing device according to some embodiments of the present disclosure;
FIG. 3 is a schematic diagram of hardware and/or software components of an exemplary mobile device according to some embodiments of the present disclosure;
FIG. 4A is a structural block diagram of a convolutional neural network for processing a two-dimensional image in the prior art;
FIG. 4B is a structural block diagram of a convolutional neural network for processing a three-dimensional image in the prior art;
FIG. 5 is a block diagram of an exemplary processing device according to some embodiments of the present disclosure;
FIG. 6 is a flowchart illustrating an exemplary process for image processing according to some embodiments of the present disclosure;
FIG. 7 is a flowchart illustrating an exemplary process for determining a plurality of to-be-processed images according to some embodiments of the present disclosure;
FIG. 8 is a flowchart illustrating an exemplary process for image processing according to some embodiments of the present disclosure;
FIG. 9 is a flowchart illustrating an exemplary process for image processing according to some embodiments of the present disclosure;
FIG. 10 is a flowchart illustrating an exemplary process for medical image processing according to some embodiments of the present disclosure;
FIG. 11 is a flowchart illustrating an exemplary process for constructing a corresponding first image with three dimensions according to some embodiments of the present disclosure;
FIG. 12 is an exemplary structural block diagram of a neural network model according to some embodiments of the present disclosure; and
FIG. 13 is an exemplary structural block diagram of a neural network model according to some embodiments of the present disclosure.

### DETAILED DESCRIPTION

In order to illustrate the technical solutions of the embodiments of the present disclosure more clearly, the following may briefly introduce the drawings used in the descriptions of the embodiments. Apparently, for those skilled in the art, the present disclosure may be practiced without the details described. In other cases, well-known methods, procedures, systems, components, and/or circuits have been described generally at a relatively high level in order to avoid unnecessarily obscuring aspects of the present disclosure. Various modifications to the embodiments disclosed in the present disclosure may be apparent to those skilled in the art, and the general principles defined in the present disclosure may be applied to other embodiments and application scenarios without departing from the spirit and scope of the present disclosure. Thus, the present disclosure may not be limited to the embodiments shown, but may be accorded the widest scope consistent with the scope of the present disclosure.

The terms used in the present disclosure may be only used to describe specific exemplary embodiments and may not limit the scope of the present disclosure. As used herein, the singular forms "a," "an," and "the" may be intended to include the plural forms as well, unless the context clearly indicates otherwise. It may be further understood that the terms "include" and/or "comprise," when used in this disclosure, specify the presence of stated features, integers, steps, operations, assemblies, and/or components, but may not exclude the presence or addition of one or more other features, integers, steps, operations, assemblies, components, and/or combination thereof.

It may be understood that the terms "system," "unit," "module," and/or "block" used herein are one method to distinguish different components, elements, sections, parts, or assemblies of different levels in ascending order. However, the terms may be displaced by other expressions if they may achieve the same purpose.

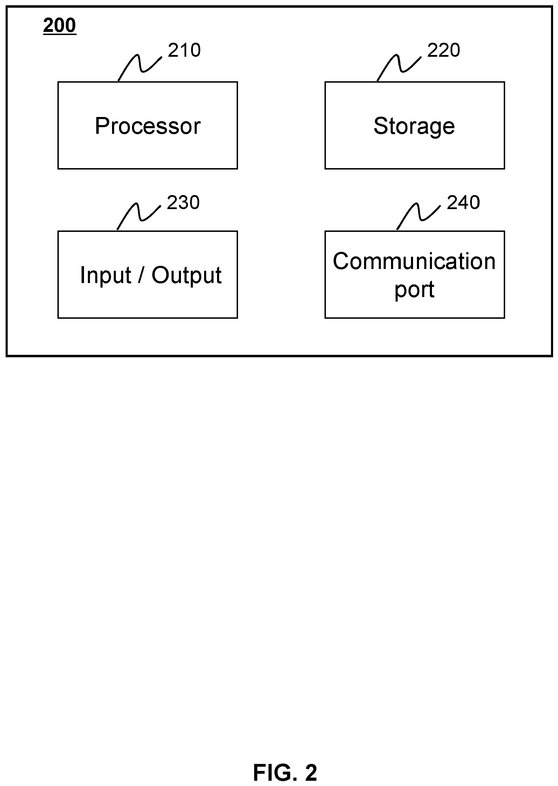

Generally, "module," "unit," or "block" as used in the present disclosure may refer to a collection of logic or software instructions stored in hardware or firmware. The modules, units, or blocks described in the present disclosure may be implemented by software and/or hardware, and may also be stored in any kind of computer-readable non-transitory medium or another storage device. In some embodiments, software modules/units/blocks may be compiled and linked into an executable program. The software module here may respond to information communicated by itself or other modules/units/blocks, and/or may respond when certain events or interruptions are detected. Software modules/units/blocks configured to perform operations on a computing device (e.g., processor 210, as shown in FIG. 2) may be provided on a computer-readable medium, such as an optical disk, a digital disc, a flash drive, a magnetic disk, or any other kind of tangible media as a digital download (initially stored in a compressed or installable format that needs to be installed, decompressed, or decrypted before an execution). The software codes herein may be stored, in part or in whole, in a storage device of a computing device which performs the operations, and used in the operation of the computing device. Software instructions may be embedded in firmware, such as an EPROM. It should be further understood that hardware modules/units/blocks may be included in connected logic components, such as gates and flip-flops, and/or may be included in programmable units, such as programmable gate arrays or processors. The modules/units/blocks or computing device functions described in the present disclosure may be preferably implemented as software modules but may also be represented in hardware or firmware. Generally, modules/units/blocks described in the present disclosure may refer to logical modules/units/blocks that may be combined with other modules/units/blocks or divided into sub-modules/ sub-unit s/sub-blocks, regardless of their physical organization or storage.

It may be understood that when a unit, an engine, a module, or a block is referred to as being "on," "connected to," or "coupled to" another unit, engine, module, or block, it may be directly on, connected or coupled to another unit, engine, module or block, or in communication with another unit, engine, module or block, or intervening another unit, engine, module or block to communicate. In the present disclosure, the term "and/or" may include any one or combinations of the above associated listed items.

Flowcharts are used in the present disclosure to illustrate operations performed by a system according to embodiments of the present disclosure. It should be understood that the preceding or following operations may not be necessarily performed in the exact order. Instead, the operations may be processed in reverse order or concurrently. At the same time, other operations may be added to these procedures, or a step or steps may be removed from these procedures.

Other features, operation methods, functions, and economic structure of related components described in the present disclosure may become more apparent from the following descriptions of the drawings, which form a part of the present disclosure. The present disclosure may provide systems and assemblies for medical imaging and/or medical treatment, such as systems and assemblies for purposes of disease diagnosis, treatment, or research. In some embodiments, the medical system may include an imaging system. The imaging system may include a single-modality imaging system and/or a multi-modality imaging system. As used herein, the term "modality" refers to an imaging or treatment method or technique that acquires, generates, processes, and/or analyzes imaging information of an object or treats the object. The single-modality system may include, for example, a computed tomography (CT) system, an X-ray imaging system, a digital radiography (DR) system, a magnetic resonance imaging (MRI) system, a positron emission tomography (PET) system, a single-photon emission computed tomography (SPECT) system, an optical coherence tomography (OCT) system, an ultrasound (US) system, a near infrared spectrum instrument (NIRS) system, or the like, or any combination thereof. The multi-modality system may include, for example, a positron emission tomography-computed tomography (PET-CT) system, a positron emission tomography-magnetic resonance imaging (PET-MRI) system, a computed tomography-magnetic resonance imaging (CT-MRI) system, a single-photon emission computed tomography-magnetic resonance imaging (SPECT-MRI) system, a digital subtraction angiography-magnetic resonance imaging (DSA-MRI) system, or the like, or any combination thereof. In some embodiments, the computed tomography (CT) system may include a C-arm system using X-rays, a dental CT or a CT system using other types of radiation, or the like.

In some embodiments, the medical system may include a treatment system. The treatment system may include a treatment planning system (TPS), an image-guided radiation therapy (IGRT), or the like. The image-guided radiation therapy (IGRT) may include a treatment device and an imaging device. The treatment device may include a linear accelerator, a cyclotron, a synchrotron, etc., which may be configured to provide a radiation therapy to a subject. The treatment device may include accelerators with various particle species, such as photons, electrons, protons, or heavy ions. The imaging device may include an MRI scanner, a CT scanner (e.g., a cone beam computed tomography (CBCT) scanner), a digital radiology (DR) scanner, an electronic portal imaging device (EPID), or the like. The medical system described below may be provided for illustration only and do not limit the scope of the present disclosure.

In the present disclosure, the subject may include an organism and/or non-organism. The organism may be a human, animal, plant, or a specific part, organ, and/or tissue thereof. For example, the subject may include the head, neck, chest, lungs, heart, stomach, blood vessels, soft tissue, tumors, nodules, or the like, or any combination thereof. In some embodiments, the subject may be an artificial composition of organic and/or inorganic matter that is animate or inanimate. In the present disclosure, the terms "object" or " subject" may be used interchangeably.

In the present disclosure, a representation of an object (e.g., a patient, a subject, or a portion thereof) in an image may be referred to simply as an object. For example, representations of organs and/or tissues (e.g., heart, liver, lungs) in an image may be referred to simply as organs or tissues. An image, including a representation of an object, may simply refer to an image of an object or an image including an object. Operations on a representation of an object in an image may refer to operations on an object. For example, a segmentation of a representation of an organ and/or a tissue that is included in a portion of an image may simply refer to a segmentation of an organ and/or a tissue. In the present disclosure, a two-dimensional image may refer to an image containing information of two dimensions (e.g., height, width). A three-dimensional image may refer to a picture containing information of three dimensions (e.g., length, width, depth).

Some embodiments of the present disclosure may relate to an image processing system and method. The image processing system and method may obtain an initial image (such as a two-dimensional image) and determine a plurality of to-be-processed images related to the initial image. Each of the plurality of to-be-processed images may be a two-dimensional image and the plurality of to-be-processed images may correspond to a three-dimensional image. A target image (a two-dimensional image) may be obtained by inputting the plurality of to-be-processed images into a processing model (such as a convolutional neural network model). The quality of the target image may be better than the initial image.

In some embodiments, a two-dimensional convolution processing may be performed on the plurality of to-be-processed images in a plurality of channels of the processing model, respectively and the target image may be obtained by performing a linear processing on a result of the two-dimensional convolution processing. Information in the plurality of to-be-processed images may be associated by performing the linear processing on the result of the two-dimensional convolution processing. Compared to the initial image, the target image may contain finer structural information. In addition, compared to using only a three-dimensional convolution to process a three-dimensional image, using the plurality of channels and the two-dimensional convolution to process the plurality of to-be-processed images (which may be equivalent to a three-dimensional image) may increase the processing speed.

In some embodiments, a preset times (e.g.,1, 2, 3) of the three-dimensional convolution processing may be performed on the plurality of to-be-processed images. Then the above-mentioned two-dimensional convolution processing and the linear processing may be performed. By performing the preset times of the three-dimensional convolution processing, the associated information of the plurality of to-be-processed images may be better extracted, and the quality of the target image may be further improved. Besides, compared to using only a three-dimensional convolution to process a three-dimensional image (with a large times of convolutions), the processing speed may be accelerated.

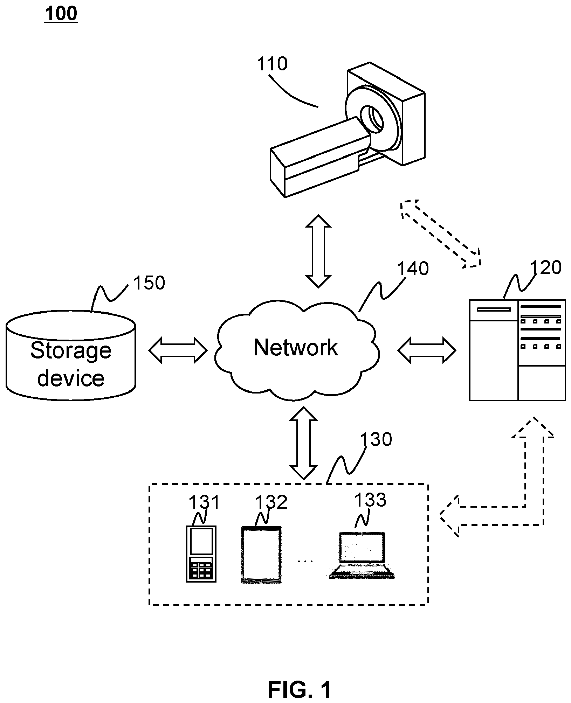

FIG. 1 is a schematic diagram of an exemplary image processing system according to some embodiments of the present disclosure. As shown in FIG. 1, the image processing system 100 may include an imaging device 110, a processing device 120, a terminal device 130, a network 140, and a storage device 150. Various components in the image processing system 100 may be connected in various ways. For example, the imaging device 110 and the processing device 120 may be connected through the network 140 or directly connected (as shown by the dotted arrow connecting the imaging device 110 and the processing device 120 in FIG. 1). As another example, the storage device 150 and the processing device 120 may be directly connected or connected through the network 140. As a further example, the terminal device 130 and the processing device 120 may be connected through the network 140 or directly connected (as shown by the dotted arrow connecting the terminal device 130 and the processing device 120 in FIG. 1).

The imaging device 110 may scan an object located within a scan area and generate imaging data (also referred to as "scan data") related to the object. The object may include a biological object (e.g., a human, an animal, etc.), a non-biological object (e.g., a phantom), or the like. In some embodiments, the imaging device 110 may be a computed tomography (CT) device, a positron emission tomography (PET) device, a magnetic resonance imaging (MRI) device, a single-photon emission computed tomography (SPECT) device, an ultrasound (US) device, a digital X-ray (DR) device, or the like, or any combination thereof (e.g., a PET-CT device, a PET-MRI device, etc.).

The processing device 120 may process data and/or information obtained from the imaging device 110, the terminal device 130, and/or the storage device 150. For example, the processing device 120 may determine, based on an initial image, a plurality of to-be-processed images related to the initial image. As another example, the processing device 120 may process the plurality of to-be-processed images based on a processing model to obtain a target image. In some embodiments, the processing device 120 may be a single server or a group server. The group server may be centralized or distributed. In some embodiments, the processing device 120 may be local or remote.

The terminal device 130 may include a mobile device 131, a tablet computer 132, a laptop computer 133, or the like, or any combination thereof. In some embodiments, the terminal device 130 may interact with other components in the image processing system 100 through network 140. For example, the terminal device 130 may send one or more control instructions to the imaging device 110 to control the imaging device 110 to scan the object according to instructions. In some embodiments, the mobile device 131 may include but be not limited to, a smartphone, a handheld game player, smart glasses, a smart watch, a wearable device, a virtual reality device, a display enhancement device, or the like, or any combination thereof. In some embodiments, the terminal device 130 may be a part of the processing device 120. In some embodiments, the terminal device 130 may be integrated with the processing device 120 as a console of the imaging device 110. For example, a user/an operator (e.g., a doctor) of the image processing system 100 may control the operation of the imaging device 110 through the console, e.g., to scan the object.

The network 140 may include any suitable network capable of facilitating the exchange of information and/or data of the image processing system 100. In some embodiments, the network 140 may include one or more network access points. For example, the network 140 may include wired and/or wireless network access points, such as base stations and/or Internet exchange points, through which one or more components of the image processing system 100 may connect to network 140 to exchange the data and/or information.

The storage device 150 may store data (e.g., scan data of an object), instructions, and/or any other information. In some embodiments, the storage device 150 may store data obtained from the imaging device 110, the terminal device 130, and/or the processing device 120. For example, the storage device 150 may store scan data obtained from the imaging device 110, or the like. In some embodiments, the storage device 150 may store data and/or instructions that may be executed or used by the processing device 120 to perform exemplary methods described in the present disclosure. In some embodiments, the storage device 150 may include a mass storage, a removable storage, a volatile read-write memory, a read-only memory (ROM), or the like, or any combination thereof. In some embodiments, the storage device 150 may be implemented through a cloud platform.

In some embodiments, the storage device 150 may be connected to the network 140 to implement the communication between one or more components in the image processing system 100 (e.g., the processing device 120, the terminal device 130, etc.). The one or more components in the image processing system 100 may read data or instructions in the storage device 150 through the network 140. In some embodiments, the storage device 150 may be a part of the processing device 120 or be separate, directly or indirectly connected to the processing device 120.

It should be noted that the above descriptions of the image processing system 100 may be provided for the purposes of illustration, and be not intended to limit the scope of the present disclosure. For those skilled in the art, multiple variations and modifications may be made based on the descriptions of the present disclosure. For example, the image processing system 100 may include one or more additional components, and/or one or more components of the image processing system 100 described above may be omitted. As another example, two or more components of the image processing system 100 may be integrated into a single component. A component of the image processing system 100 may be implemented on two or more subcomponents.

FIG. 2 is a schematic diagram of hardware and/or software components of an exemplary computing device according to some embodiments of the present disclosure. In some embodiments, one or more components of the image processing system 100 (e.g., the processing device 120) may be implemented on a computing device 200.

As shown in FIG. 2, the computing device 200 may include a processor 210, a storage 220, an input/output (I/O) 230, and a communication port 240.

The processor 210 may execute computer instructions (e.g., program codes) and perform the functions of the processing device 120 according to the technique described in the present disclosure. The computer instructions may include performing specific functions described in the present disclosure, for example, routines, programs, components, signals, parts, data structures, procedures, modules, and functions. For example, the processor 210 may obtain an initial image from the terminal device 130 and/or the storage device 150. In some embodiments, the processor 210 may include one or more hardware processors.

For illustration purposes, only one processor is illustrated in the computing device 200. However, it should be noted that the computing device 200 in the present disclosure may also include a plurality of processors, and thus, the operations and/or methods described in the present disclosure to be performed by one processor may also be performed jointly or separately by the plurality of processors. For example, if operation A and operation B are performed in the processor of computing device 200 of the present disclosure, it should be understood that operation A and operation B may also be performed by two different processors jointly or separately in the computing device 200 (e.g., a first processor may perform operation A, a second processor may perform operation B, or the first processor and the second processor may jointly perform operations A and B).

The storage 220 may store data/information obtained from the imaging device 110, the processing device 120, the storage device 150, the terminal device 130, and/or any other components of the image processing system 100. In some embodiments, the storage 220 may store one or more programs and/or instructions to perform the exemplary methods described in the present disclosure.

The input/output 230 may input and/or output signals, data, information, or the like. In some embodiments, the input/output 230 may implement interaction between a user and the processing device 120. In some embodiments, the input/output 230 may include an input device and an output device.

The communication port 240 may connect to a network (e.g., the network 140) to facilitate data communication. The communication port 240 may establish a connection between the processing device 120 and the imaging device 110, the terminal device 130, and/or the storage device 150. The connection may be wired, wireless, or a combination of both to implement data transmission and data reception. In some embodiments, the communication port 240 may be and/or include a standardized communication port, such as RS232, RS485, or the like. In some embodiments, the communication port 240 may be a specially designed port. For example, the communication port 240 may be designed according to the Digital Imaging and Communications in Medicine (DICOM) protocol.

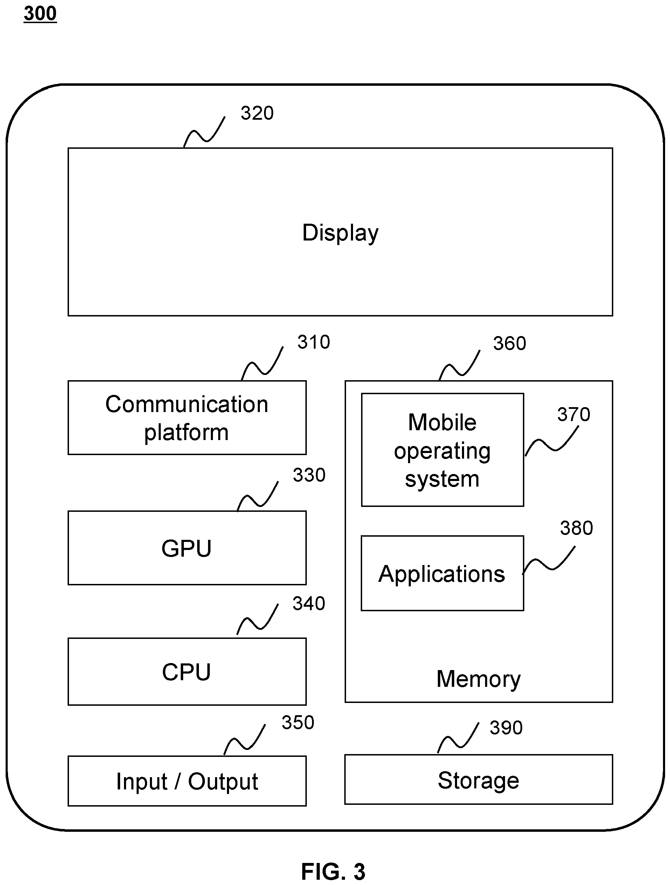

FIG. 3 is a schematic diagram of hardware and/or software components of an exemplary mobile device according to some embodiments of the present disclosure. In some embodiments, one or more components of the image processing system 100 (e.g., the terminal device 130 and/or the processing device 120) may be implemented on a mobile device 300.

As shown in FIG. 3, the mobile device 300 may include a communication platform 310, a display 320, a graphic processing unit (GPU) 330, a central processing unit (CPU) 340, an input/output 350, a memory 360, and a storage 390. In some embodiments, any other suitable components, including but being not limited to a system bus or a controller (not shown), may also be included within the mobile device 300. In some embodiments, a mobile operating system 370 (e.g., iOS^{™}, Android^{™}, Windows Phone^{™}) and one or more applications 380 may be loaded from the storage 390 into the memory 360 for execution by the CPU 340. The applications 380 may include a browser or any other suitable mobile applications for receiving and presenting information related to image processing or other information from the processing device 120. The interaction between the user and the information stream may be implemented based on the input/output 350 and provided to the processing device 120 and/or other components of the image processing system 100 through the network 140.

In order to implement the various modules, units, and functions described in the present disclosure, a computer hardware platform may be used as a hardware platform for one or more components described in the present disclosure. The hardware elements, operating systems, and programming languages of such computers may be conventional, and it may be assumed that those skilled in the art are sufficiently familiar with these hardware elements, operating systems, and programming languages to adapt these techniques to the image processing described herein. A computer with user interface components may be used to implement a personal computer (PC) or any other type of workstation or terminal device. If programmed properly, a computer may also be used as a server. It is believed that those skilled in the art are familiar with the structures, programs, and general operations of such computing devices. Therefore, no descriptions of the drawings are required.

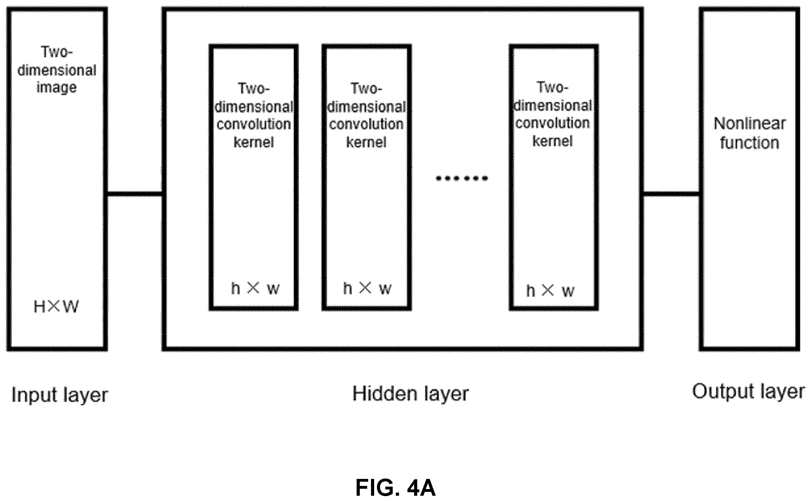

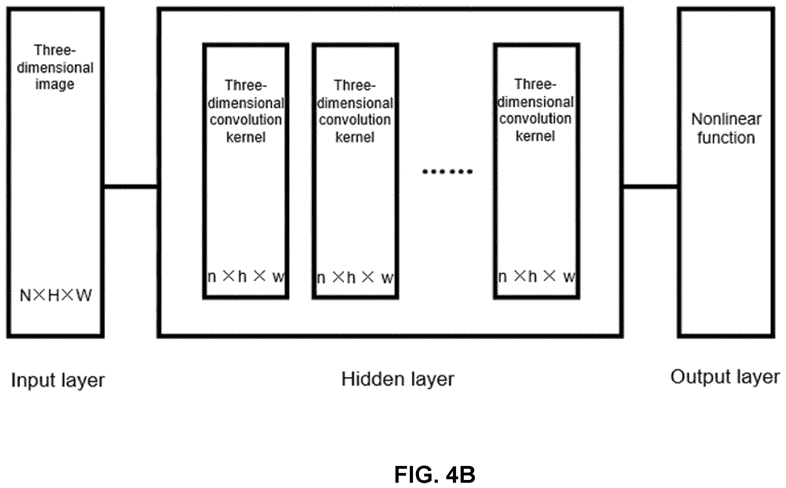

FIG. 4A is a structural block diagram of a convolutional neural network for processing a two-dimensional image in the prior art. FIG. 4B is a structural block diagram of a convolutional neural network for processing a three-dimensional image in the prior art.

As shown in FIG. 4A, an existing convolutional neural network (CNN) for processing a two-dimensional image may include an input layer, a hidden layer, and an output layer. The input layer may be configured to input the two-dimensional image. The hidden layer may be configured to perform a convolution processing on the inputted two-dimensional image. The convolution processing may use a two-dimensional convolution kernel. The output layer may be configured to process (e.g., via a nonlinear function the convolved two-dimensional image to generate an output (e.g., a two-dimensional image) of the convolutional neural networks.

As shown in FIG. 4B, an existing convolutional neural network for processing a three-dimensional image may include an input layer, a hidden layer, and an output layer. The input layer may be configured to input the three-dimensional image. The hidden layer may be configured to perform a convolution processing on the inputted three-dimensional image. The convolution processing may use a three-dimensional convolution kernel. The output layer may be configured to process (e.g., via a nonlinear function) the convolved three-dimensional image to generate an output (e.g., a three-dimensional image) of the convolutional neural networks.

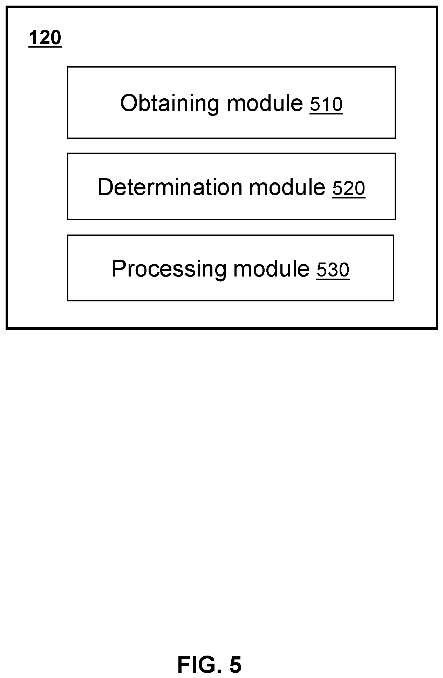

FIG. 5 is a block diagram of an exemplary processing device according to some embodiments of the present disclosure. The processing device 120 may include an obtaining module 510, a determination module 520, and a processing module 530.

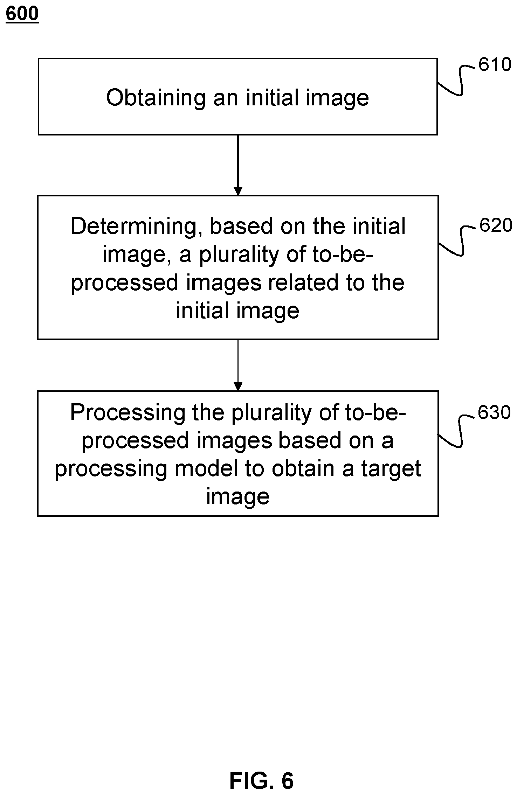

The obtaining module 510 may be configured to obtain an initial image. In some embodiments, the initial image may include at least a portion of an object (e.g., human, animal), such as a head, spine, neck, chest, lung, heart, stomach, blood vessel, soft tissue, tumor, nodule, or the like. In some embodiments, the initial image may be a two-dimensional image. For more descriptions of the initial images, please refer to FIGs. 6-13 and related descriptions thereof.

The determination module 520 may be configured to determine, based on the initial image, a plurality of to-be-processed images (e.g., two-dimensional images) related to the initial image. In some embodiments, the plurality of to-be-processed images may include information of the initial image. For example, the plurality of to-be-processed images may include the initial image or a portion of the initial image. As another example, the initial image may be directly used as one of the plurality of to-be-processed images. In some embodiments, regions of interest in at least two of the plurality of to-be-processed images (which include the initial image or a portion thereof) may have a similar feature, such as structural information, texture information, or the like. The "similar" used here may refer to a similarity between features of different regions of interest (such as texture information, gradient information, grayscale information) that exceeds a threshold.

In some embodiments, the determination module 520 may obtain at least two additional images. For example, regions of interest contained in the at least two additional images may be the same as a region of interest contained in the initial image. The determination module 520 may determine the initial image and at least one of the at least two additional images as the plurality of to-be-processed images. For example, the determination module 520 may select a plurality of consecutive images adjacent to the initial image as the plurality of to-be-processed images.

In some embodiments, the plurality of to-be-processed images may be generated based on the initial image. For example, the determination module 520 may obtain a plurality of image blocks based on the initial image and/or at least one of the at least two additional images. The plurality of image blocks may have image regions with a same or similar feature, that is, the plurality of image blocks may be related. For example, the plurality of image blocks may have a structural continuity. "Similar" used here may refer to a similarity between features (such as texture information, gradient information, grayscale information) of different image blocks exceeding a threshold. In some embodiments, the image regions may correspond to an organ of the object (e.g., a brain, a spine) or a portion of an organ (e.g., a brain tissue, a cervical spine, a thoracic spine, a lumbar spine). In some embodiments, the determination module 520 may determine image regions containing a same or similar feature according to image features of the initial image and/or at least one of the at least two additional images to determine the plurality of image blocks. "Similar" used here may refer to a similarity between features (such as texture information, gradient information, grayscale information) of different image regions exceeding a threshold. The determination module 520 may obtain the plurality of to-be-processed images based on the plurality of image blocks. For more descriptions about the plurality of to-be-processed images, please refer to FIGs. 6-13 and related descriptions thereof.

The processing module 530 may be configured to process the plurality of to-be-processed images based on a processing model to obtain a target image (e.g., a two-dimensional image). In some embodiments, the processing module 530 may perform a three-dimensional convolution processing, a matrix transformation processing, a two-dimensional convolution processing, and a linear processing using the processing model to obtain the target image. In some embodiments, the processing module 530 may perform the two-dimensional convolution processing (but not the three-dimensional convolution processing) and the linear processing using the processing model. For more descriptions of the processing model and the target image, please refer to FIGs. 6-13 and related descriptions thereof.

In some embodiments, the processing device 120 may include a training module (not shown in the drawings). The training module may be configured to train the processing model. The training module may train a preliminary model based on a large number of training samples to obtain the processing model. It should be noted that the training module may also be configured on other processing devices so that the model training and model usage may be performed on different processing devices.

It should be noted that the above is provided for the purpose of illustration and is not intended to limit the scope of the present disclosure. For those skilled in the art, multiple variations and modifications may be made based on the descriptions of the present disclosure. However, such variations and modifications do not depart from the scope of the present disclosure. Modules in the processing device 120 may connect or communicate with each other via a wired or wireless connection. Two or more modules may be combined into one module, and any module may be divided into two or more units. For example, the processing device 120 may further include a storage module (not shown in FIG. 5). The storage module may be configured to store data generated during any process performed by any component of the processing device 120. As another example, each component of the processing device 120 may include a storage device. As another example, components of the processing device 120 may share a common storage device. As another example, the training module may be unnecessary, and the processing model may be obtained from a storage device disclosed elsewhere in the present disclosure (e.g., the storage device 150, the storage 220, the storage 390).

FIG. 6 is a flowchart illustrating an exemplary process for image processing according to some embodiments of the present disclosure. In some embodiments, process 600 may be implemented in the image processing system 100 shown in FIG. 1. For example, process 600 may be stored in a storage medium (e.g., the storage device 150 or the storage 220 of the processing device 120) in the form of instructions and may be invoked and/or executed by the processing device 120 (e.g., the processor 210 of the processing device 120 or one or more modules in the processing device 120 shown in FIG. 5). Operations of process 600 presented below may be for the purpose of illustration. In some embodiments, process 600 may be accomplished based on one or more additional operations not described and/or without one or more operations discussed in the present disclosure. Additionally, the order of operations of process 600 shown in FIG. 6 and described below, may be not intended to be limiting.

In 610, the processing device 120 (e.g., the obtaining module 510) may obtain an initial image. In some embodiments, the initial image may include at least a portion of an object (e.g., human, animal), e.g., a head, spine, neck, chest, lung, heart, stomach, blood vessel, soft tissue, tumor, nodule, or the like. In some embodiments, the initial image may be a two-dimensional image. In some embodiments, the initial image may include a computed tomography (CT) image, a nuclear magnetic resonance (MR) image, a positron emission tomography (PET) image, an ultrasound image, an X-ray image, a single-photon emission computed tomography (SPECT) image, or the like, or any combination thereof.

In some embodiments, the processing device 120 may obtain the initial image from an imaging device (e.g., the imaging device 110), a storage device (e.g., the storage device 150, the storage 220, the storage 390), or an external device connected to the image processing system 100 (e.g., an external storage). In some embodiments, the processing device 120 may instruct the imaging device (e.g., the imaging device 110) to scan the object to obtain imaging data and generate the initial image based on the imaging data.

In some embodiments, a scan protocol may be set up firstly, the imaging data may be acquired based on the imaging device, and then the imaging data may be reconstructed based on reconstruction parameters to generate an image sequence. The processing device 120 may select one image in the image sequence as the initial image. In some embodiments, images with different layer thicknesses and layer spacing may be obtained by adjusting parameters in the scanning protocol. The processing device 120 may select one image among the images with different layer thicknesses and layer spacing as the initial image. In some embodiments, the reconstruction parameters may be adjusted based on an image generated based on a scan. For example, a region of interest (e.g., a lesion) of the object may be determined based on the image generated based on the scan. If the region of interest is not in a reconstruction center, the reconstruction parameters may be adjusted to allow the region of interest in the center, and a new image may be obtained by performing a new scan.

In 620, the processing device 120 (e.g., the determination module 520) may determine, based on the initial image, a plurality of to-be-processed images related to the initial image. In some embodiments, the plurality of to-be-processed images may be acquired and generated by one or more imaging devices. In some embodiments, the imaging device used to acquire the plurality of to-be-processed images may be the same as or different from the imaging device used to acquire the initial image. In some embodiments, at least two of the plurality of to-be-processed images may be generated based on data acquired by imaging devices of different modalities. Merely by way of example, the plurality of to-be-processed images may include a computed tomography (CT) image, a nuclear magnetic resonance (MR) image, a positron emission computed tomography (PET) image, an ultrasound image, an X-ray image, a single-photon emission computed tomography (SPECT) image, or the like, or any combination thereof.

In some embodiments, the plurality of to-be-processed images may include information of the initial image. For example, the plurality of to-be-processed images may include the initial image or a portion of the initial image. As another example, the initial image may be directly used as one of the plurality of to-be-processed images. In some embodiments, regions of interest in at least two (which include the initial image or a portion thereof) of the plurality of to-be-processed images may have a similar feature, such as structural information, texture information, or the like.

In some embodiments, each of the plurality of to-be-processed images may be a two-dimensional image. In some embodiments, the plurality of to-be-processed images may include regions of interest at a plurality of angles. Correspondingly, the plurality of to-be-processed images may also be understood to correspond to a three-dimensional image. In some embodiments, sizes of the plurality of to-be-processed images may be the same (e.g., 50*50) or different.

In some embodiments, the processing device 120 may obtain at least two additional images. For example, regions of interest contained in the at least two additional images may be the same as the region of interest contained in the initial image. The processing device 120 may take the initial image and at least one of the at least two additional images as the plurality of to-be-processed images. For example, the processing device 120 may obtain an image sequence (which includes the initial image). The processing device 120 may select a plurality of consecutive images adjacent to the initial image from the image sequence, such as spatially and/or temporally adjacent to the initial image, as the plurality of to-be-processed images. In some embodiments, the at least two additional images may be generated based on data acquired by imaging devices of the same modality or different modalities. In some embodiments, the processing device 120 may perform preprocessing (e.g., normalization, noise reduction, artifact removal, brightness adjustment, contrast adjustment) on the at least two additional images and then determine the plurality of to-be-processed images.

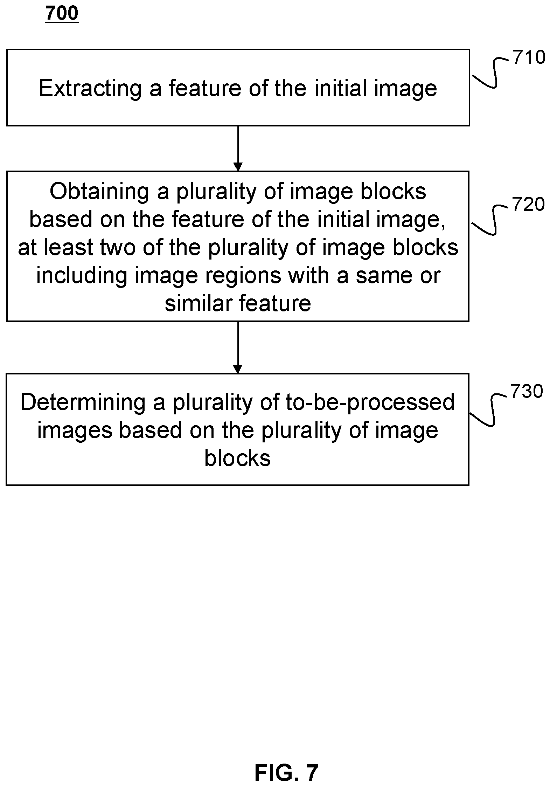

In some embodiments, the plurality of to-be-processed images may be generated based on the initial image. For example, the processing device 120 may obtain a plurality of image blocks based on the initial image and/or at least one of the at least two additional images. The plurality of image blocks may have image regions with a same or similar feature, that is, the plurality of image blocks may be related. For example, the plurality of image blocks may have a structural continuity. In some embodiments, the image regions may correspond to an organ of the object (e.g., a brain, a spine) or a portion of an organ (e.g., a brain tissue, a cervical spine, a thoracic spine, a lumbar spine). In some embodiments, the processing device 120 may determine image regions containing a same or similar feature according to image features of the initial image and/or at least one of the at least two additional images to determine the plurality of image blocks. The processing device 120 may obtain the plurality of to-be-processed images based on the plurality of image blocks. For more descriptions about image blocks determination, please refer to FIG. 7 and related descriptions thereof.

In 630, the processing device 120 (e.g., the processing module 530) may process the plurality of to-be-processed images based on a processing model to obtain a target image (e.g., a two-dimensional image). In some embodiments, the processing model may have one or more functions. The processing model may be configured to perform a three-dimensional convolution processing based on a plurality of two-dimensional images (e.g., the plurality of to-be-processed images obtained in operation 620) or a three-dimensional image. In some embodiments, a times of the three-dimensional convolution processing may be preset, such as 1 time, 2 times, 3 times. The processing model may be configured to perform a matrix transformation processing on a result of the three-dimensional convolution processing. The processing model may be configured to perform a two-dimensional convolution processing on a result of the matrix transformation processing. The processing model may be configured to perform a linear processing on a result of the two-dimensional convolution processing to obtain a two-dimensional image. In some embodiments, the processing model may include a matrix transformation module configured to perform the matrix transformation processing on the result of the three-dimensional convolution processing. Through the matrix transformation processing, the plurality of two-dimensional images or a two-dimensional image sequence may be transformed into two-dimensional images of a plurality of channels. A count of the channels may be the same as a count of the two-dimensional images or a count of two-dimensional images in the two-dimensional image sequence. In some embodiments, the processing model may include a linear processing module configured to perform the linear processing on the result of the two-dimensional convolution processing to obtain the two-dimensional image. Through the linear processing, information of the plurality of two-dimensional images may be superimposed or integrated into the two-dimensional image so that information of the resulting image may be richer (for example, the image may have more detailed structural information).

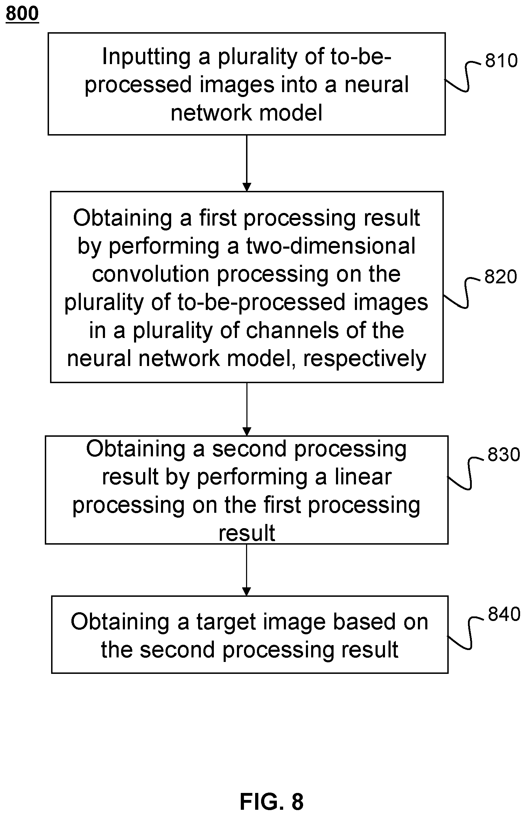

In some embodiments, the processing model may perform all or a portion of the one or more functions. In some embodiments, the processing device 120 may perform the three-dimensional convolution processing, the matrix transformation processing, the two-dimensional convolution processing, and the linear processing based on the processing model to obtain the target image. In some embodiments, the processing device 120 may perform the two-dimensional convolution processing (but not the three-dimensional convolution processing) and the linear processing based on the processing model to obtain the target image. For more related descriptions, please refer to FIGs. 8-9 and/or related descriptions thereof.

In some embodiments, the processing model may include a neural network model. Tensorflow may be used to implement the architecture of the neural network (such as Caffe and PyTorch). An exemplary neural network model may include a convolutional neural network model, a recurrent neural network (RNN) model, a generative adversarial neural network (GAN) model, a deep convolutional encoding and decoding (DCED) neural network model, a fully convolutional neural network (FCN) model, a backpropagation (BP) neural network model, a radial basis function (RBF) neural network model, a deep belief (DBN) neural network model, an Elman neural network model, or the like, or any combination thereof. An exemplary convolutional neural network model may include a space utilization-based convolutional neural network model, a depth-based convolutional neural network model, a width-based and multi-connection convolutional neural network model, or the like.

In some embodiments, the preliminary model may be trained based on a large number of training samples to obtain the processing model. Each training sample may include a training initial image, a plurality of training to-be-processed images, and a target training image (i.e., a gold standard). The large number of training samples may be input into the preliminary model in batches. Accordingly, the preliminary model may output a training result. If a difference between the training result and a gold standard exceeds a certain threshold, parameters in the preliminary model may be adjusted. The above operations may be iterated until the difference between the training result and the gold standard is less than the threshold or a count of the iterations exceeds a certain threshold, and the training may stop.

In some embodiments, the training samples may correspond to different organs of a training object. In some embodiments, a processing model for processing an organ may be trained based on training samples corresponding to the same organ. For example, the training samples may correspond to a lung, and a corresponding trained model may be configured only to process lung images. As another example, the training samples may correspond to a brain, and a corresponding trained model may be configured only to process brain images. In some embodiments, training samples corresponding to different organs may be used to train a processing model configured to process different organs. For example, the processing model may be configured to process lung images and brain images.

In some embodiments, the processing model may be pre-trained. The processing device 120 may obtain the processing model from a storage device (e.g., the storage device 150, the storage 220, the storage 390) or an external device (e.g., an external storage) connected to the image processing system 100.

In some embodiments, the target image may correspond to the initial image. In some embodiments, a position of a training initial image may be fixed in the plurality of training to-be-processed images in the training samples, such as the first position, the middle position, the last position, or the like. For the determination of the plurality of to-be-processed images in 620, the processing device 120 may arrange the initial image in the same position as above, so that the target image may correspond to the initial image, that is, the initial image may be optimized through the processing model based on the plurality of to-be-processed images. For example, the processing model may use the plurality of to-be-processed images to determine image information related to the initial image after de-noising or artifact reduction. Then, the target image may be determined by fusing the image information after the de-noising or the artifact reduction with the initial image.

In some embodiments, the quality of the target image may be better than the initial image. For example, there may be fewer artifacts or noise in the target image than in the initial image. As another example, a resolution and/or a contrast of the target image may be higher than that of the initial image. In some embodiments, the target image may include identification information related to the region of interest. The identification information related to the region of interest may include a contour of the region of interest, a location of the region of interest, a size of the region of interest, or the like, or any combination thereof.

FIG. 7 is a flowchart illustrating an exemplary process for determining a plurality of to-be-processed images according to some embodiments of the present disclosure. In some embodiments, process 700 may be implemented in the image processing system 100 shown in FIG. 1. For example, process 700 may be stored in a storage medium (e.g., the storage device 150 or the storage 220 of the processing device 120) in the form of instructions and may be invoked and/or executed by the processing device 120 (e.g., the processor 210 of the processing device 120 or one or more modules in the processing device 120 shown in FIG. 5). Operations of process 700 presented below may be for a purpose of illustration. In some embodiments, process 700 may be accomplished based on one or more additional operations not described and/or without one or more operations discussed in the present disclosure. Additionally, the order of operations of process 700 shown in FIG. 7 and described below may be not intended to be limiting.

In 710, the processing device 120 (e.g., the determination module 520) may extract a feature of an initial image. In some embodiments, the feature of the initial image may include texture information, gradient information, grayscale information, color information, or the like. In some embodiments, the processing device 120 may extract the feature of the initial image based on an image recognition algorithm.

In some embodiments, the processing device 120 may identify and/or segment a region of interest in the initial image. Then, the processing device 120 may only extract the feature of an image portion corresponding to the region of interest. For example, the processing device 120 may identify the region of interest in the initial image based on an image recognition algorithm. As another example, the processing device 120 may segment the region of interest in the initial image based on an image segmentation algorithm.

In 720, the processing device 120 (e.g., the determination module 520) may obtain a plurality of image blocks based on the feature of the initial image. At least two of the plurality of image blocks may include image regions with a same or similar feature. "Same or similar feature" may refer that a difference in structural similarities (SSIM) of different image regions is less than a first threshold or that structural similarities of different image regions is greater than a second threshold. For example, the at least two of the plurality of image blocks may include a same organ or a same portion of an organ. In some embodiments, the processing device 120 may divide the initial image (e.g., 200*200) into a plurality of image regions (50*50). The plurality of image regions may overlap or may not overlap. For example, taking a spine image as an example, the plurality of image regions may include a cervical spine image region, a thoracic spine image region, a lumbar spine image region, or the like.

In some embodiments, the processing device 120 may obtain the plurality of image blocks according to the structural similarities (SSIM) of the plurality of image regions. For example, the processing device 120 may classify image regions whose structural similarities are greater than the second threshold or a difference in the structural similarities of different image regions is less than the first threshold as the plurality of image blocks.

In some embodiments, the processing device 120 may obtain the plurality of image blocks according to grayscale information (e.g., grayscale values) of the plurality of image regions. For example, the processing device 120 may designate image regions whose gray value differences are within a certain range as the plurality of image blocks. In some embodiments, the plurality of image blocks may also be obtained by a manual operation.

In some embodiments, a size of each of the plurality of image blocks may be the same or different and be set according to specific requirements. In some embodiments, corresponding to one initial image, there may be a group of image blocks or a plurality of groups of image blocks, and each group may include a plurality of image blocks. Taking a brain image as an example, there may be a group of image blocks corresponding to a brain tissue and/or a group of image blocks corresponding to a skull. In some embodiments, image blocks generated based on the initial image may be determined as a group of image blocks, and image blocks generated based on other images related to the initial image (e.g., an image adjacent to the initial image, an image with the same scan range as the initial image but a different modality) may be determined as another group of image blocks.

In 730, the processing device 120 (e.g., the determination module 520) may determine a plurality of to-be-processed images based on the plurality of image blocks (e.g., a plurality of image blocks in one or more groups of image blocks). In some embodiments, the processing device 120 may perform a matrix transformation on each of the plurality of image blocks to determine the plurality of to-be-processed images. In some embodiments, the matrix transformation may be used to normalize each of the plurality of image blocks. In some embodiments, the matrix transformation may be used to transform each of the plurality of image blocks into an image block sequence. In some embodiments, a count of the plurality of image blocks may be the same as that of the plurality of to-be-processed images.

FIG. 8 is a flowchart illustrating an exemplary process for image processing according to some embodiments of the present disclosure. In some embodiments, process 800 may be implemented in the image processing system 100 shown in FIG. 1. For example, process 800 may be stored in a storage medium (e.g., the storage device 150 or the storage 220 of the processing device 120) in the form of instructions and may be invoked and/or executed by the processing device 120 (e.g., the processor 210 of the processing device 120 or one or more modules in the processing device 120 shown in FIG. 5). Operations of process 800 presented below may be for a purpose of illustration. In some embodiments, process 800 may be accomplished based on one or more additional operations not described and/or without one or more operations discussed in the present disclosure. Additionally, the order of operations of process 800 shown in FIG. 8 and described below may be not intended to be limiting.

In 810, the processing device 120 (e.g., the processing module 530) may input a plurality of to-be-processed images into a neural network model. In some embodiments, the processing device 120 may input the plurality of to-be-processed images into the neural network model via an input layer of the neural network model.

In 820, the processing device 120 (e.g., the processing module 530) may obtain a first processing result by performing a two-dimensional convolution processing on the plurality of to-be-processed images in a plurality of channels of the neural network model, respectively. In some embodiments, in each of the plurality of channels of the neural network model, the processing device 120 may obtain a corresponding portion of the first processing result by performing the two-dimensional convolution processing on one of the plurality of to-be-processed images.

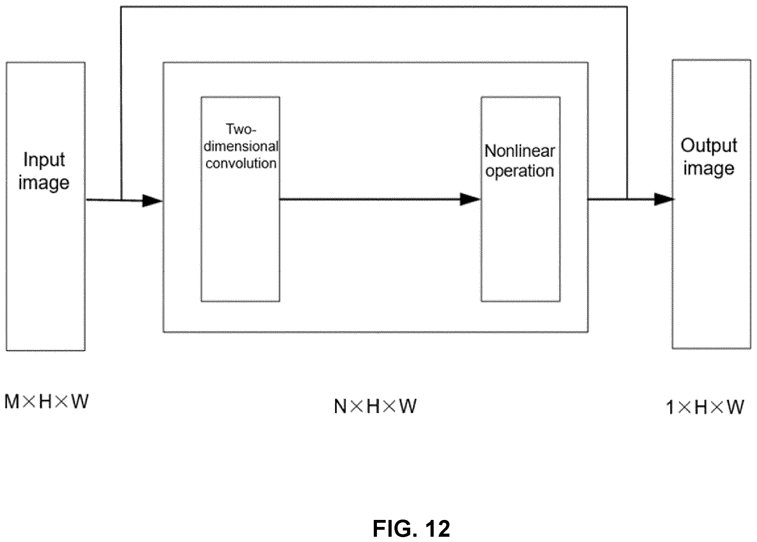

In some embodiments, taking a channel and a corresponding to-be-processed image as an example, the processing device 120 may perform the two-dimensional convolution processing on the corresponding to-be-processed image in the channel through a two-dimensional convolution kernel. It should be noted that, in addition to performing the two-dimensional convolution processing, the processing device 120 may also perform a regularization processing and/or nonlinear processing to obtain the first processing result. In some embodiments, a structure of the neural network model involved in process 800 may be shown in FIG. 12.

In some embodiments, the processing model may use other images to be processed except the initial image as reference images and process the initial images in combination with information of the reference images. The reference images may carry additional details about a structure in the initial image. Thus, more information about the structure may be displayed in a target image. For more related descriptions, please refer to operation 630 in FIG. 6 and/or related descriptions thereof.

In 830, the processing device 120 (e.g., the processing module 530) may obtain a second processing result by performing a linear processing on the first processing result. In some embodiments, the processing device 120 may assign a weight to a portion of the first processing result corresponding to each channel and then perform a weighting processing to obtain the second processing result. In some embodiments, the processing device 120 may assign a higher weight to a portion of the first processing result corresponding to the initial image. In some embodiments, operation 830 may be performed within the neural network model. For example, the neural network model may include a linear processing module configured to obtain the second processing result by performing the linear processing on the first processing result. Therefore, the target image may contain a feature of each of the plurality of to-be-processed images (such as a texture feature, a gradient feature, a grayscale feature) so that more information about the corresponding structure may be displayed in the target image. In some embodiments, operation 830 may be performed by a linear processing module outside the neural network model.

In 840, a target image may be obtained based on the second processing result. In some embodiments, if the plurality of to-be-processed images include a plurality of consecutive images adjacent to the initial image, the second processing result may be the target image. In some embodiments, if the plurality of to-be-processed images are obtained based on a plurality of image blocks, the processing device 120 may perform a fusion (e.g., stitching) on the second processing result to determine the target image. In some embodiments, if one or more image blocks corresponding to the initial image cover the entire initial image, the processing device 120 may stitch the second processing result to determine the target image. In some embodiments, if the one or more image blocks corresponding to the initial image only cover a portion of the initial image, the processing device 120 may stitch the second processing result and set pixel values of an uncovered portion of the initial image to a preset value (e.g., 0) to determine the target image. In some embodiments, the processing device 120 may fuse the second processing result with the initial image to determine the target image.

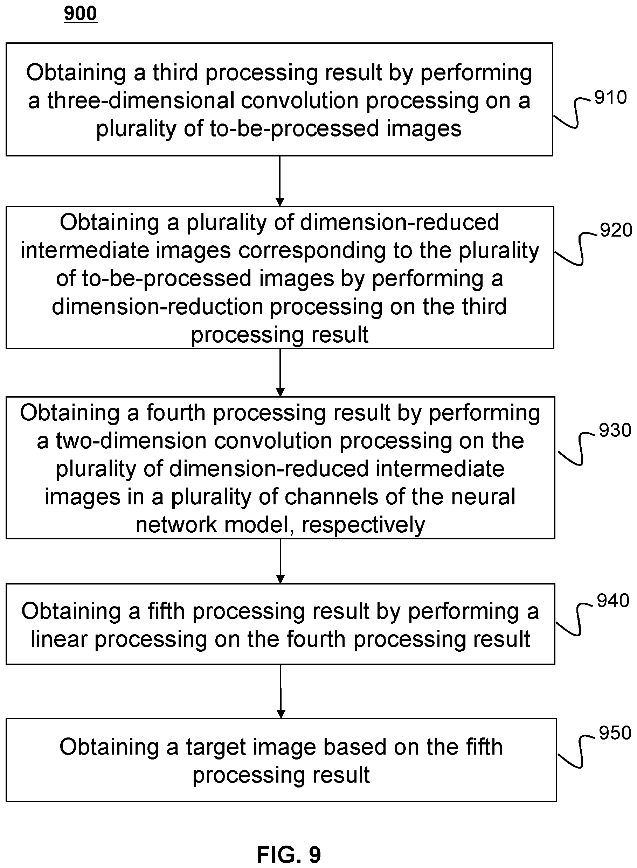

FIG. 9 is a flowchart illustrating an exemplary process for image processing according to some embodiments of the present disclosure. In some embodiments, process 900 may be implemented in the image processing system 100 shown in FIG. 1. For example, process 900 may be stored in a storage medium (e.g., the storage device 150 or the storage 220 of the processing device 120) in the form of instructions and may be invoked and/or executed by the processing device 120 (e.g., the processor 210 of the processing device 120 or one or more modules in the processing device 120 shown in FIG. 5). Operations of process 900 presented below may be intended for a purpose of illustration. In some embodiments, process 900 may be accomplished based on one or more additional operations not described and/or without one or more operations discussed in the present disclosure. Additionally, the order of operations of process 900 shown in FIG. 9 and described below may be not intended to be limiting.

In 910, the processing device 120 (e.g., the processing module 530) may obtain a third processing result by performing a three-dimensional convolution processing on a plurality of to-be-processed images. Through the three-dimensional convolution processing, associated information between the plurality of to-be-processed images may be extracted (for example, associated spatial information of a structure of interest). In some embodiments, the third processing result may include the associated information and the plurality of to-be-processed images. In some embodiments, a times of three-dimensional convolution processing may be preset, such as 1 time, 2 times, 3 times. In some embodiments, the preset times may be an empirical value.

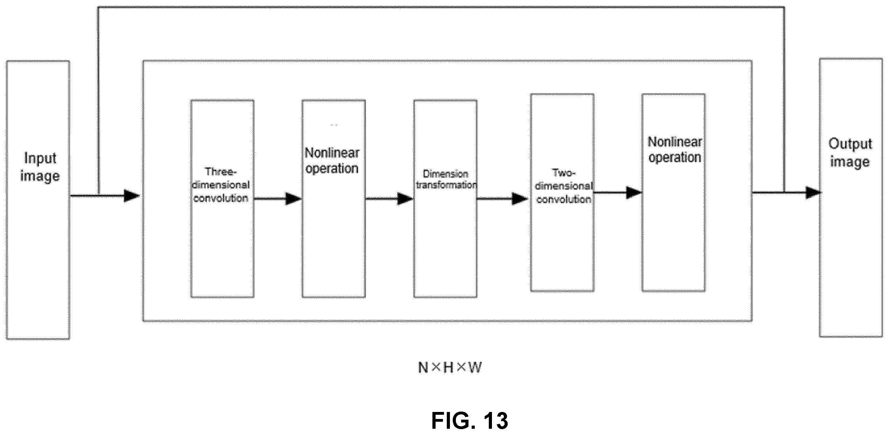

It should be noted that, in addition to performing the three-dimensional convolution processing, the processing device 120 may also perform a regularization processing and/or a nonlinear processing to obtain the third processing result. In some embodiments, a structure of the neural network model involved in process 900 may be shown in FIG. 13.

In 920, the processing device 120 (e.g., the processing module 530) may obtain a plurality of dimension-reduced intermediate images corresponding to the plurality of to-be-processed images by performing a dimension-reduction processing on the third processing result. In some embodiments, the processing device 120 may perform a dimension-reduction processing by performing a matrix transformation through a matrix transformation module in the neural network model.

In 930, the processing device 120 (e.g., the processing module 530) may obtain a fourth processing result by performing a two-dimension convolution processing on the plurality of dimension-reduced intermediate images in a plurality of channels of the neural network model, respectively. In some embodiments, the process for obtaining the fourth processing result may be the same as or similar to the process for obtaining the first processing result in operation 820. For more related descriptions, please refer to the descriptions in operation 820.

In 940, the processing device 120 (e.g., the processing module 530) may obtain a fifth processing result by performing a linear processing on the fourth processing result. In some embodiments, the process for obtaining the fifth processing result may be the same as or similar to the process for obtaining the second processing result in operation 830. For more related descriptions, please refer to the descriptions in operation 830.

In 950, the processing device 120 (e.g., the processing module 530) may obtain a target image based on the fifth processing result. In some embodiments, the process for obtaining the target image in operation 950 may be the same as or similar to the process for obtaining the target image in operation 840. For more related descriptions, please refer to the descriptions in operation 840.

As mentioned above, the convolutional neural network model may perform a convolution processing (such as a two-dimensional convolution and a three-dimensional convolution), a nonlinear processing (such as via an activation function), a normalized processing, a linear processing, or the like, on input images (that is, the plurality of to-be-processed images). The order of the processing may be adjusted in various ways, which may not constitute a limitation on the present disclosure. For example, the order of the processing may be the convolution processing, the normalization processing, the nonlinear processing, and the linear processing. As another example, the order of the processing may be the convolution processing, the linear processing, the normalization processing, and the nonlinear processing.

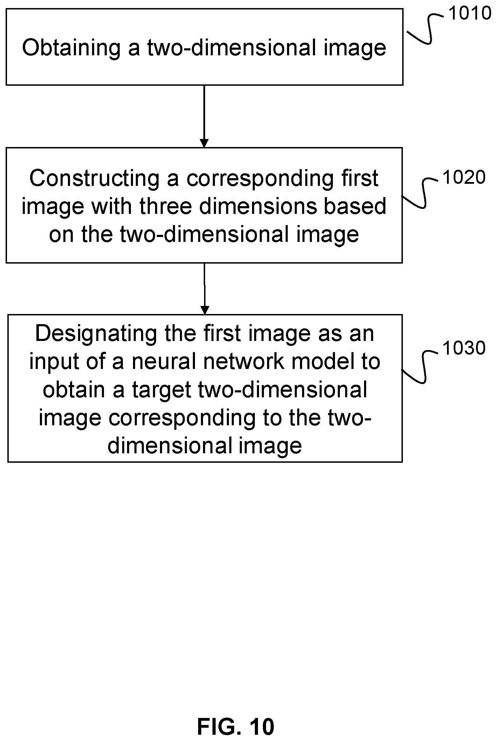

FIG. 10 is a flowchart illustrating an exemplary process for medical image processing according to some embodiments of the present disclosure. As shown in FIG. 10, the medical image processing may include operations 1010-1030.

In 1010, a two-dimensional image may be obtained.

In a clinical scanning protocol, an operator may reconstruct images with different layer thicknesses and layer spacing under the same scanning protocol. During the scanning process, the operator may adjust a reconstruction parameter, such as a position of a reconstruction center in a reconstruction protocol according to a preview image of the reconstructed image. Correspondingly, one scanning protocol may contain a sequence of reconstructed medical images, and a plurality of scanning protocols may contain a plurality of sequences of reconstructed medical images. Each medical image sequence may contain one or more consecutive two-dimensional images. The one or more consecutive two-dimensional images may be understood as a plurality of two-dimensional images arranged according to a reconstruction order or may be a plurality of two-dimensional images arranged according to reconstruction regions. These different medical image sequences may differ in reconstruction parameters, such as a layer thickness and layer spacing. The present disclosure may not limit the process for obtaining the medical image sequence. In a practical application, one of the two-dimensional images in the medical image sequence may be first obtained for processing.

The two-dimensional image in operation 1010 may also refer to an initial image. For more descriptions of the obtaining the two-dimensional image, please refer to operation 610 in FIG. 6 and related descriptions thereof.

In 1020, a corresponding first image with three dimensions may be constructed based on the two-dimensional image.

The first image with three dimensions may be understood as a three-dimensional image or a two-dimensional image with third dimension information. The third dimension information may be depth information. The two-dimensional image with the third dimension information may be a two-dimensional image sequence. When each two-dimensional image is processed, a corresponding first image with three dimensions may be constructed for each two-dimensional image so as to add more reference information (for example, the same or similar feature of the same or similar structure contained in different two-dimensional images) to obtain a finer structure of the two-dimensional image.

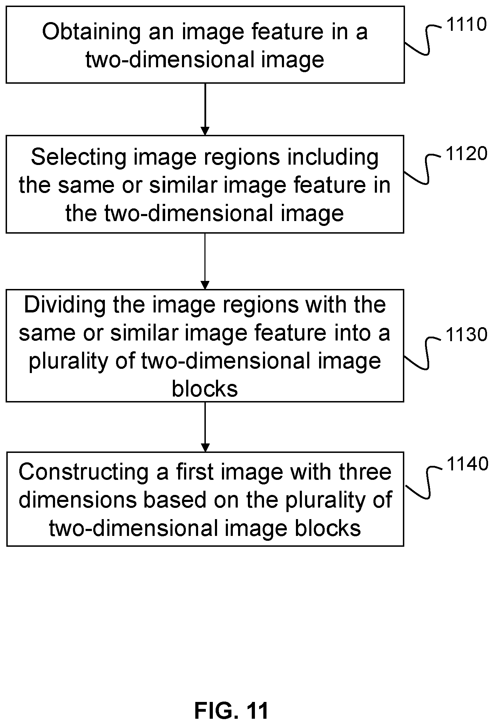

The first image with three dimensions in operation 1020 may also refer to a plurality of to-be-processed images. For more descriptions on constructing the first image with three dimensions, please refer to FIG. 11 and related descriptions thereof.

In 1030, the first image may be designated as an input of a neural network model to obtain a target two-dimensional image corresponding to the two-dimensional image. The neural network model in operation 1030 may also refer to as a processing model, and the target two-dimensional image may also refer to as a target image. For more descriptions of obtaining the target two-dimensional image, please refer to operation 630 in FIG. 6 and related descriptions thereof.

The image processing process provided in the present disclosure may modify an original neural network structure, superimpose the target two-dimensional image into an output of the neural network, which may introduce additional third-dimensional information of the two-dimensional image, and use a two-dimensional convolution for a network forward propagation. Compared with an existing two-dimensional convolutional neural network, which processes one two-dimensional image at a time, one dimension (such as depth information) may be added, which may be equivalent to adding one piece of information, thereby improving the image resolution. Compared with an existing three-dimensional convolutional neural network, which directly performs multiple times of three-dimensional convolution processing on the entire image sequence, the process provided in the present disclosure may not significantly reduce the speed of image processing since only a portion of the calculation amount of the three-dimensional convolution is increased. The problem of incompatibility between the image processing accuracy and the processing speed may be solved, and the effect of improving the image processing accuracy without significantly reducing the image processing speed may be achieved.

In some embodiments, the constructing the corresponding first image with three dimensions based on the two-dimensional image may include:
obtaining a plurality of two-dimensional images adjacent to the two-dimensional image, and constructing the corresponding first image with three dimensions based on the plurality of two-dimensional images. Specifically, the plurality of two-dimensional images adjacent to the two-dimensional image may be selected from an image sequence. For more descriptions, please refer to operation 620 and related descriptions thereof.

For CT scanning, a medical image sequence may include two-dimensional images reconstructed from a plurality of planes, and each of the two-dimensional images may include different feature information of a scanned region. When the scanned region is diagnosed, since a lesion portion changes continuously, it may not be possible to accurately and comprehensively observe the structure of the lesion based only on one of the two-dimensional images. In some embodiments, the plurality of two-dimensional images adjacent to the two-dimensional image may be selected from the medical image sequence. The corresponding first image with three dimensions may be constructed according to the plurality of two-dimensional images so as to add additional feature information (such as texture information, gradient information, grayscale information of the structure). The scanned region may be diagnosed based on information contained in the plurality of two-dimensional images, thereby improving the ability to distinguish the lesion.

FIG. 11 is a flowchart illustrating an exemplary process for constructing a corresponding first image with three dimensions according to some embodiments of the present disclosure. As shown in FIG. 11, the constructing a corresponding first image with three dimensions based on the two-dimensional image may include operations 1110-1140.

In 1110, an image feature in a two-dimensional image may be obtained.

Specifically, a region of interest in the two-dimensional image, that is, a region that needs to be observed, may be identified through a medical image recognition algorithm, and then an identified region may be segmented from the two-dimensional image based on an image segmentation algorithm.

In medical images, texture features of different medical images may be also different. For example, a texture feature in a scanning image of a skull may be fewer, while a texture feature in a scanning image of an abdominal cavity may be relatively more. In addition, a directional feature of the texture in the medical image may also represent different portions corresponding to the medical image to a certain extent. Therefore, in this embodiment, the texture feature may be used as the image feature in the two-dimensional image. Of course, the image feature may also include gradient information, grayscale information, color information, etc., of an image. The two-dimensional image may be divided into a plurality of image regions according to the image feature. In some embodiments, the plurality of image regions may be first divided based on the image feature. In some embodiments, the plurality of image regions may be first divided, and then the image feature of each of the plurality of image regions may be obtained.

In 1120, image regions including the same or similar image feature in the two-dimensional image may be selected.

For example, the two-dimensional image may be an image obtained by scanning a human spine, and the two-dimensional image may be divided into a cervical spine image region, a thoracic spine image region, and a lumbar spine image region according to the image feature. Each of the cervical spine image region, the thoracic spine image region, and the lumbar spine image region may include the similar image feature.

In 1130, the image regions with the same or similar image feature may be divided into a plurality of two-dimensional image blocks. In some embodiments, each image region may be determined as an image block. In some embodiments, each image region may be further divided into a plurality of sub-image regions, and each sub-image region may be determined as an image block.

It may be understood that the plurality of two-dimensional image blocks may be related. The plurality of two-dimensional image blocks may have a structural continuity.

In 1140, a first image with three dimensions may be constructed based on the plurality of two-dimensional image blocks.

Specifically, the plurality of two-dimensional images may be obtained by performing a matrix transformation on each two-dimensional image block. The first image with three dimensions may then be constructed based on the plurality of two-dimensional images. Taking a spine image region as an example, a size of a cervical spine image region may be 200*200. The cervical spine image region with the size of 200*200 may be divided into a plurality of (for example, 16) two-dimensional image blocks with a size of 50*50. Then, the matrix transformation may be performed on the 16 two-dimensional image blocks to obtain 16 two-dimensional cervical spine images with the size of 50*50. A three-dimensional cervical spine image may be constructed based on the 16 two-dimensional cervical spine images with the size of 50*50 so that the cervical spine of a human body may be better observed. It should be noted that a count of the two-dimensional image block may be other values, such as 8, 32, and so on. The size of the two-dimensional image block may be other values, such as 32*32, 64*64, 128*128, etc.

For more descriptions about operations 1110-1140, please refer to FIG. 7 and related descriptions thereof.

FIG. 12 is an exemplary structural block diagram of a neural network model according to some embodiments of the present disclosure. As shown in FIG 12, the designating the first image as an input of the neural network model to obtain a target two-dimensional image corresponding to the two-dimensional image may include:
using an increased dimension of the first image with respect to the two-dimensional image as a channel; and/or
performing a two-dimensional convolution processing and a nonlinear processing on the first image to obtain the target two-dimensional image corresponding to the two-dimensional image. For the process for obtaining the target two-dimensional image corresponding to the two-dimensional image (which may also refer to the target image), please refer to FIG. 8 and related descriptions thereof.

The neural network in the present disclosure may include any artificial neural network that may implement a deep learning algorithm. In the artificial neural network, a Convolutional Neural Network (CNN) may be a type of a Feedforward Neural Network including a convolutional computation, having a deep structure, and being one of representative algorithms of deep learning. The deep learning neural network may have a capacity of memory, parameter sharing, unlimited storage, etc., so it may learn a nonlinear feature of image noise with high efficiency. The deep learning neural network may have been proven and successfully implemented in applications related to data detection. Most of noise in the medical image may come from a random noise source, which may be a typical nonlinear noise. Therefore, in some embodiments of the present disclosure, the deep learning neural network may be used to perform a noise reduction processing on the medical image to achieve a better result.

The present disclosure may use TensorFlow to implement a structure of the neural network, and the optional structure may include Caffe, PyTorch, etc. In some embodiments, the convolutional neural network (CNN) may be used as an example to describe the structure of the neural network used in the embodiment of the present disclosure. In other embodiments, a Recurrent Neural Network (RNN) may also be used, which may be not specifically limited in the embodiment. The convolutional neural network may include an input layer, a hidden layer, and an output layer. The input layer may be configured for data input. In some embodiments, the input of the input layer may be the first image with three dimensions. The hidden layer may include a convolutional layer and a pooling layer. The convolutional layer may be configured to extract different features in the first image. A low-level convolutional layer may extract some lower-level features, and a higher-level convolutional layer may iteratively extract more complex features from the low-level features. The pooling layer may be configured to perform a nonlinear processing, reduce a count of model parameters, and reduce an overfitting problem. In some other embodiments, the hidden layer may further include a normalization layer configured to forcibly pull an input distribution, which gradually approaches a limit saturation region of a value interval after mapping to a nonlinear transformation function, to a standard normal distribution with mean 0 and variance 1, making the input value of the nonlinear transformation function fall into a region that is more sensitive to the input, so as to avoid the problem of gradient disappearance.

The output layer may be used to output the two-dimensional image after the input image is processed.

In some embodiments, an increased dimension of the first image with respect to the two-dimensional image may be used as a channel. For example, a size of the two-dimensional image may be H*W, wherein H is a height of the two-dimensional image, W is a width of the two-dimensional image. The constructed first image may be M*H*W, wherein M is the increased dimension. In some embodiments, the increased dimension may be used as channel information to process the first image.

In some embodiments, the increased dimension may be used as channel information, and the constructed first image may be understood as M two-dimensional images with a size of H*W. The colors of the M two-dimensional images may be different and/or may contain different image features. Different two-dimensional convolution processing may be performed on different image features. Finally, the target two-dimensional image corresponding to the two-dimensional image may be obtained. The two-dimensional convolution processing may achieve a data enrichment processing on the input data (also known as the result of the two-dimensional convolution processing, such as the feature information of an extracted image) and then combine them to output the target two-dimensional image. If the count of channels input in the two-dimensional convolution is 3, that is, the dimension of the input data may be 3*H*W. In the process of convolution, the image dimension may be N*H*W, wherein N changes constantly. Usually, in the process of initial convolution, N may be continuously increased first, and then as the convolution is performed, N may be continuously decreased. For example, N may be increased from 3 to 16, then increased to 64, then decreased from 64 to 32, 16, and finally to 1. A filter (that is, a convolution kernel) and the image data may be convolved separately in the channel direction. The convolved values may be added together, and after a processing, such as a nonlinear processing, the target two-dimensional image of 1*H*W may finally be output. In some embodiments, in the process of convolution, the dimension of the image may remain 3*H*W, the two-dimensional convolution may be performed on the image of each channel separately and a linear processing may be performed on the processing result of the image of each channel, and the target two-dimensional image may be superimposed as 1*H*W.

In some embodiments, after inputting the first image with three dimensions corresponding to the two-dimensional image into the neural network model, the neural network model may use other two-dimensional images except the two-dimensional image as reference images and may combine information of the reference images to process the two-dimensional image. For example, after constructing the first image with three dimensions based on (M-1) two-dimensional images of a size of H*W adjacent to the two-dimensional to-be-processed image, the first image may be understood as M two-dimensional images with a size of H^{∗}W. The (M-1) two-dimensional images with the size of H^{∗}W adjacent to the two-dimensional to-be-processed image may be used as the reference images, and the reference images may carry more detailed information of the two-dimensional to-be-processed image. By combining the reference images adjacent to the two-dimensional to-be-processed image, a finer structure of the two-dimensional to-be-processed image may be obtained.

FIG. 13 is an exemplary structural block diagram of a neural network model according to some embodiments of the present disclosure. As shown in FIG. 13, the designating the first image as an input of the neural network model to obtain a target two-dimensional image corresponding to the two-dimensional image may include:
determining an increased dimension of the first image relative to the two-dimensional image as an image dimension;
performing a three-dimensional convolution processing and a nonlinear processing on the first image;
performing a dimension transformation processing on data obtained after the processing, and determining the increased dimension as a channel; and
obtaining a target two-dimensional image corresponding to the two-dimensional image by performing a two-dimensional convolution processing, a nonlinear processing, and/or a linear processing on the first image after the dimension transformation processing. For more related descriptions, please refer to FIG. 9 and related descriptions thereof.

In some embodiments, the increased dimension may be determined as the image dimension, that is, the constructed first image may be regarded as a three-dimensional image. A traditional method for a three-dimensional image may include a processing by a three-dimensional convolutional neural network. The three-dimensional convolutional neural network may directly perform a convolution on the three-dimensional image to extract a three-dimensional spatial feature of the image. In the present disclosure, in a three-dimensional image formed by a plurality of two-dimensional images, a three-dimensional convolution kernel filter may be used to perform a preset count of three-dimensional convolution processing on the three-dimensional image to extract spatial information in the image. Specifically, in a first convolution layer of the three-dimensional convolutional neural network model, a preset first convolution kernel (for example, a three-dimensional convolution kernel) may be used to perform a convolution processing on the first image, and a preset activation function may be used to non-linearly map the first image to obtain a first-level feature diagram. In a plurality of convolution layers after the first layer, a second convolution kernel (such as a three-dimensional convolution kernel) may be used to convolve the first-level feature diagram, respectively, and a preset activation function may be used to non-linearly map the first image to obtain a target-level feature diagram. A second target-level feature diagram may be obtained by performing a dimension-reduction processing on the first target-level feature diagram. The dimension of the second target-level feature diagram may be the same as the dimension of the first-level feature diagram. A target feature diagram may be obtained by linearly combining the first-level feature diagram and the second target-level feature diagram. Then a dimension transformation may be performed to transform the increased dimension into channel information. The target two-dimensional image corresponding to the two-dimensional image may be obtained by performing a two-dimensional convolution processing, a nonlinear operation processing, and/or a linear processing on a changed target feature diagram. It may be understood that, in some embodiments, after performing the three-dimensional convolution processing and the nonlinear processing on the first image, the dimension transformation processing may be performed, and the increased dimension may be determined as the channel information. The subsequent execution process may be similar to the processing above process for designating the first image as the input of the neural network model, and details may be not repeated in the embodiment.

It should be noted that, in some embodiments, the increased dimension of the first image with respect to the two-dimensional image may be determined as the image dimension. A preset count of the three-dimensional convolution processing may be performed on the first image. The preset count may be one time, two times, three times, or the like. The specific count may be not limited, but the count of the processing through the three-dimensional convolutional neural network may be as small as possible so that the processing speed may be improved.

In some embodiments, the increased dimension of the first image with respect to the two-dimensional image may be determined as the image dimension. A preset count of the three-dimensional convolution processing and the nonlinear processing may be performed on the first image, and then the dimension transformation processing may be performed. The increased dimension may be determined as the channel information. The two-dimensional convolutional neural network model may be used for processing to obtain the target two-dimensional image corresponding to the two-dimensional image, which may input more information into the network and ensure the image processing speed.

It may be understood that only a processing result of a two-dimensional image in a medical image may be obtained through the above processing process, and a medical image sequence may include a plurality of two-dimensional images. Therefore, the above processing operations may need to be performed cyclically to obtain the target two-dimensional image corresponding to each of the plurality of two-dimensional images in the medical image sequence.

The above-mentioned medical image processing may include other processing such as noise reduction processing, artifact removal processing, etc., which may be not limited in some embodiments.

In some embodiments, the medical image may include a CT image, an MR image, a PET image, or the like.

The present disclosure may take a noise reduction processing of a CT image as an example for descriptions. A computed tomography (CT) device may generally include a gantry, a scanning bed, and a console for a doctor to operate. One side of the gantry may be provided with a tube, and a side opposite to the tube may be provided with a detector. The console may be a computer device that controls scanning. The computer device may also be used to receive scan data acquired by the detector, process the data, and finally form a CT image. When the scanning is performed based on CT, a patient may lie on the scanning bed. The scanning bed may transport the patient into a bore of the gantry. The tube arranged on the gantry may emit X-rays. The X-rays may pass through the patient and be received by the detector to generate the scan data. The scan data may be transmitted to the computer device. The computer device may perform a preprocessing and/or image reconstruction on the scan data to obtain the CT image.

Due to the radiation damage of the X-rays to the human body, the dose of the CT scan should be as low as possible. When the dose of CT decreases, a count of photons reaching the detector may also decrease, so the image may show obvious noise. Noise may cover a lesion, making it more difficult for the doctor to diagnose the lesion. Therefore, how to reduce the noise of the image while reducing the CT dose is an important issue in the field of CT. Neural networks have achieved very good results in the field of noise reduction. However, traditional noise reduction methods based on noise reduction neural networks have limited improvement in lesion distinction, which limits the clinical application of the noise reduction neural networks.

The present disclosure may construct a corresponding first image with three dimensions according to a two-dimensional image. A first image may be used as an input of a neural network model, and a target two-dimensional image corresponding to the two-dimensional image may be obtained. On one aspect, the method provided in the present disclosure may introduce additional third-dimensional information of the two-dimensional image and use a two-dimensional convolution for a network forward propagation. Compared with an existing two-dimensional convolutional neural network, which processes one two-dimensional image at a time, the image processing accuracy may be improved. Compared with an existing three-dimensional convolutional neural network, which directly performs a plurality of three-dimensional convolution processing on an entire image sequence, an overall network calculation amount may be reduced, the speed of image processing may be improved, and the problem in the prior art of incompatibility between the image processing accuracy and the processing speed may be solved.

Some embodiments of the present disclosure may further provide an image processing device. The device may include a processor and a storage. The storage may be used for storing instructions. The processor may be used for executing the instructions, so as to implement the image processing method described in any embodiment of the present disclosure.

Some embodiments of the present disclosure may further provide a computer-readable storage medium. The computer-readable storage medium may store computer instructions. When the computer instructions are executed by the processor, operations corresponding to the image processing method described in any embodiment of the present disclosure may be implemented.

The basic concepts have been described above. Obviously, for those skilled in the art after reading the present disclosure, the above disclosure may be only an example and may not constitute a limitation to the present disclosure. Although not explicitly described herein, various modifications, improvements, and corrections to the present disclosure may be performed by those skilled in the art. Such modifications, improvements, and corrections may be suggested in the present disclosure, so such modifications, improvements, and corrections may still belong to the spirit and scope of the exemplary embodiments of the present disclosure.

Meanwhile, the present disclosure may use specific words to describe the embodiments of the present disclosure. For example, "one embodiment," "an embodiment," and/or "some embodiments" may mean a certain feature, structure, or characteristic associated with at least one embodiment of the present disclosure. Therefore, it should be emphasized and noted that two or more references to "an embodiment" or "one embodiment" or "an alternative embodiment" in different places in the present disclosure may be not necessarily referring to the same embodiment. Furthermore, certain features, structures or characteristics of the one or more embodiments of the present disclosure may be combined as appropriate.

Furthermore, those skilled in the art may appreciate that aspects of the present disclosure may be illustrated and described in several patentable classes or situations, including any new and useful process, machine, product, matter, or any combination thereof, or any new and useful improvements to it. Accordingly, various aspects of the present disclosure may be performed entirely by a hardware, entirely by a software (including a firmware, a resident software, a microcode, etc.), or by a combination of a hardware and a software. The above hardware or software may be referred to as a "unit", "module" or "system". Furthermore, the aspects of the present disclosure may take the form of a computer program product embodied in one or more computer-readable media, and the product may include a computer-readable program code.

A computer-readable signal medium may contain a propagated data signal with the computer program code embodied therein, for example, at baseband or as part of a carrier wave. Such propagating signals may take a variety of forms, including electromagnetic, optical, etc., or any suitable combination. The computer-readable signal medium may be any computer-readable medium other than a computer-readable storage medium that may communicate, propagate, or transmit a program for use by coupling to an instruction execution system, apparatus, or device. A program code on the computer-readable signal medium may be propagated by any suitable medium, including radio, cable, fiber optic cable, RF, etc., or any combinations of the foregoing.

The computer program code required for the operation of the various parts of the present disclosure may be written in any one or more programming languages. The program code may run entirely on a user's computer, or as a stand-alone software package on the user's computer, or partly on the user's computer and partly on a remote computer, or entirely on a remote computer or server. In the latter case, the remote computer may be connected to the user's computer through any network, such as a local area network (LAN) or a wide area network (WAN), or to an external computer (e.g., through the Internet), or in a cloud computing environment, or as a service to use, e.g., software as a service (SaaS).

Furthermore, unless explicitly stated in the claims, the order of processing elements and sequences described in the present disclosure, the use of alphanumerics, or the use of other names may be not intended to limit the order of the processes and methods of the present disclosure. While the preceding disclosure discusses some embodiments of the invention that are presently believed to be helpful, it is to be understood that such details may be only for purposes of illustration and that the appended claims may be not limited to the disclosed embodiments. On the contrary, the claims may be intended to cover all modifications and equivalent combinations that come within the spirit and scope of the embodiments of the present disclosure. For example, although the implementation of various components described above may be embodied in a hardware device, it may only be implemented through a solution of a software. For example, a described system may be installed on an existing sever or a mobile device.

Similarly, it should be noted that, in order to simplify the expressions disclosed in the present disclosure and thereby help the understanding of one or more embodiments of the invention, in the preceding descriptions of the embodiments of the present disclosure, various features may sometimes be combined into one embodiment, in one drawing or descriptions thereof. However, this method for disclosure may not imply that an object of the present disclosure requires more features than are recited in the claims. In fact, there may be fewer features of an embodiment than all features of a single embodiment disclosed above.

In some embodiments, numbers representing quantities/properties used to describe and claim certain embodiments of the present disclosure may be understood as modified by the terms "about", "approximately" or "substantially" in some cases. Unless stated otherwise, "about", "approximately" or "substantially" may mean that a variation of ±20% may be allowed for the stated number. Accordingly, in some embodiments, numerical parameters used in the present disclosure and claims may be approximate values, which may be changed according to the features required by individual embodiments. In some embodiments, the numerical parameters may consider specified significant digits and adopt a method for general digit reservation. Although the numerical fields and parameters used to confirm a range breadth in some embodiments of the present disclosure may be approximate values, in specific embodiments, such values may be set as accurately as possible within a feasible range.

For each patent, patent application, patent application publication, or other materials (such as articles, books, specifications, publications, documents, events and/or similar things) cited in the present disclosure may be hereby incorporated into the present disclosure as a reference. Any indictment documentation relating to the aforementioned documents, any such document inconsistent with or conflicting with the present disclosure, or any such document limiting the broad scope of the claims to which the present disclosure relates sooner or later may be also excluded. For example, if there is any inconsistency or conflict between the descriptions, definition, and/or use of terms in the auxiliary materials of the present disclosure and the content of the present disclosure, the descriptions, definition, and/or use of terms in the present disclosure may be subject to the present disclosure.

Finally, it should be understood that the embodiments described in the present disclosure may only used to illustrate principles of the embodiments of the present disclosure. Other variations may also belong to the scope of the present disclosure. Therefore, as an example and not a limitation, alternative configurations of the embodiments of the present disclosure may be regarded as consistent with the teaching of the present disclosure. Accordingly, the embodiments of the present disclosure may not limited to the embodiments introduced and described in the present disclosure explicitly.

## Claims

1. A system, comprising:
at least one storage device including a set of instructions; and
at least one processor in communication with the at least one storage device, wherein, when executing the set of instructions, the at least one processor is configured to cause the system to perform operations including:
obtaining an initial image;
determining, based on the initial image, a plurality of to-be-processed images related to the initial image; and
processing the plurality of to-be-processed images based on a processing model to obtain a target image of the initial image.

2. The system of claim 1, wherein the initial image is a two-dimensional image, the plurality of to-be-processed images include information of the initial image, or the target image is a two-dimensional image.

3. The system of claim 1, wherein each of the plurality of to-be-processed images is a two-dimensional image, or the plurality of to-be-processed images correspond to a three-dimensional image.

4. The system of claim 1, wherein a similarity of structure information or texture information of regions of interest in at least two of the plurality of to-be-processed images exceeds a threshold.

5. The system of claim 1, wherein at least two of the plurality of to-be-processed images are generated based on data acquired by imaging devices of different modalities.

6. The system of claim 1, wherein the target image includes identification information related to a region of interest, and the identification information related to the region of interest includes a contour of the region of interest, a location of the region of interest, or a size of the region of interest.

7. The system of claim 1, wherein the determining, based on the initial image, the plurality of to-be-processed images related to the initial image includes:
obtaining at least two additional images; and
determining the initial image and at least one of the at least two additional images as the plurality of to-be-processed images.

8. The system of claim 1, wherein the determining, based on the initial image, the plurality of to-be-processed images related to the initial image includes:
selecting a plurality of consecutive images adjacent to the initial image as the plurality of to-be-processed images.

9. The system of claim 1, wherein the determining, based on the initial image, the plurality of to-be-processed images related to the initial image includes:
extracting a feature of the initial image;
obtaining a plurality of image blocks based on the feature of the initial image, wherein at least two of the plurality of image blocks include image regions with a same feature; and
determining the plurality of to-be-processed images based on the plurality of image blocks.

10. The system of claim 9, wherein the determining the plurality of to-be-processed images based on the plurality of image blocks includes:
determining the plurality of to-be-processed images by performing a matrix transformation on each of the plurality of image blocks.

11. The system of claim 1, wherein the processing model includes a neural network model.

12. The system of claim 11, wherein the neural network model includes a matrix transformation module, and the neural network model is configured to:
perform a three-dimensional convolution processing based on a plurality of two-dimensional images or a three-dimensional image;
perform a matrix transformation processing on a result of the three-dimensional convolution processing;
perform a two-dimensional convolution processing on a result of the matrix transformation processing; and
obtain a two-dimensional image by performing a linear processing on a result of the two-dimensional convolution processing.

13. The system of claim 11, wherein the processing the plurality of to-be-processed images based on the processing model to obtain the target image includes:
inputting the plurality of to-be-processed images into the neural network model;
obtaining a first processing result by performing a two-dimensional convolution processing on the plurality of to-be-processed images in a plurality of channels of the neural network model, respectively;
obtaining a second processing result by performing a linear processing on the first processing result; and
obtaining the target image based on the second processing result.

14. The system of claim 13, wherein the obtaining the first processing result by performing the two-dimensional convolution processing on the plurality of to-be-processed images in the plurality of channels of the neural network model, respectively, includes:
in each of the plurality of channels of the neural network model, obtaining a corresponding portion of the first processing result by performing the two-dimensional convolution processing on one of the plurality of to-be-processed images.

15. The system of claim 13, wherein the plurality of to-be-processed images include a plurality of image blocks, and the obtaining the target image based on the second processing result includes:
fusing the second processing result to determine the target image, or fusing the second processing result and the initial image to determine the target image.

16. The system of claim 11, wherein the processing the plurality of to-be-processed images based on the processing model to obtain the target image includes:
obtaining a third processing result by performing a three-dimensional convolution processing on the plurality of to-be-processed images;
obtaining a plurality of dimension-reduced intermediate images corresponding to the plurality of to-be-processed images by performing a dimension-reduction processing on the third processing result;
obtaining a fourth processing result by performing a two-dimension convolution processing on the plurality of dimension-reduced intermediate images in a plurality of channels of the neural network model, respectively;
obtaining a fifth processing result by performing a linear processing on the fourth processing result; and
obtaining the target image based on the fifth processing result.

17. The system of claim 1, wherein the initial image, the plurality of to-be-processed images, or the target image includes at least one of a computed tomography image, a nuclear magnetic resonance image, a positron emission computed tomography image, or an ultrasound image.

18. A method implemented on a computing device including at least one processor and at least one storage device, comprising:
obtaining an initial image;
determining, based on the initial image, a plurality of to-be-processed images related to the initial image; and
processing the plurality of to-be-processed images based on a processing model to obtain a target image.

19. A non-transitory computer-readable medium including executable instructions, wherein when executed by at least one processor, the executable instructions direct the at least one processor to perform a method, and the method includes:
obtaining an initial image;
determining, based on the initial image, a plurality of to-be-processed images related to the initial image; and
processing the plurality of to-be-processed images based on a processing model to obtain a target image.

20. A system, comprising:
an obtaining module configured to obtain an initial image;
a determination module configured to determine, based on the initial image, a plurality of to-be-processed images related to the initial image; and
a processing module configured to process the plurality of to-be-processed images based on a processing model to obtain a target image.
